# EUROPÄISCHE PATENTSCHRIFT

(11) **EP 2 697 390 B1**
(45) Veröffentlichungstag und Bekanntmachung des Hinweises auf die Patenterteilung: **12.08.2020**
(21) Anmeldenummer: 12715930.9
(22) Anmeldetag: 13.04.2012
(51) Int. Cl.: C12Q 1/68, C12Q 1/6806, G01N 33/569, G01N 1/30

(54) **AUTOMATISIERTER NACHWEIS VON ZIRKULIERENDEN TUMORZELLEN**
AUTOMATED DETECTION OF CIRCULATING TUMOR CELLS
DÉTECTION AUTOMATISÉE DE CELLULES TUMORALES CIRCULANTES

(30) Priorität: 20.05.2011 DE 102011076221
(43) Veröffentlichungstag der Anmeldung: 19.02.2014
(73) Patentinhaber: Siemens Healthcare GmbH, 91052 Erlangen (DE)
(72) Erfinder: FRIEDRICH, Katja, 63906 Erlenbach am Main (DE); BANGERT, Joachim, 91052 Erlangen (DE); GUMBRECHT, Walter, 91074 Herzogenaurach (DE); HILTAWSKY, Karsten, 58239 Schwerte (DE); PAULICKA, Peter, 91341 Röttenbach (DE); STANZEL, Manfred, 92334 Berching (DE)
(74) Vertreter: Schweitzer, Klaus
(86) Internationale Anmeldenummer: PCT/EP2012/056802
(87) Internationale Veröffentlichungsnummer: WO 2012/159820

(56) Entgegenhaltungen:
- EP-A1- 0 713 087
- WO-A2-03/022999
- US-A- 3 731 806
- US-A- 4 124 449
- US-A1- 2011 076 684

## Beschreibung

Die Erfindung ist auf dem Gebiet der in vitro Diagnostik und betrifft ein Verfahren und eine Anordnung zum Nachweis lebender, zirkulierender oder disseminierter Zellen aus Körperflüssigkeiten (z.B. Blut, Urin) bzw. mit Flüssigkeit versetzter Gewebeproben (z.B. Knochenmark). Das erfindungsgemäße Verfahren dient insbesondere zur automatisierten Gewinnung und zur Analyse von zirkulierenden Tumorzellen und findet somit bevorzugt Anwendung in der Tumordiagnostik.

Die Erfindung ermöglicht den automatisierten Nachweis von Zellen oder Zellbestandteilen aus peripherem Blut oder Knochenmark durch einen funktionellen Test, nachdem das Blut oder Knochenmark durch ein spezielles Filtrationsverfahren gefiltert wurde. Bei den Zellen handelt es sich insbesondere um zirkulierende Tumorzellen (engl. circulating tumor cells, CTC oder (plur.) CTCs), mesenchymale Stammzellen aus peripherem Blut oder Bakterien aus Blut oder anderen Körperflüssigkeiten sowie disseminierte Tumorzellen aus Knochenmark (engl. disseminated tumor cells, DTC).

Das Vorkommen von CTCs in peripherem Blut ist ein Hinweis auf eine mögliche Streuung von Zellen eines soliden Tumors zu einem sehr frühen Stadium, in dem mit üblichen bildgebenden Untersuchungsverfahren noch keine Metastasierung nachgewiesen werden kann. Daher stellen sowohl der Nachweis als auch die Charakterisierung von CTCs in peripherem Blut vielversprechende Möglichkeiten dar, systemische Tumorzellausbreitung sehr frühzeitig zu erkennen und CTCs als prognostischen Marker zu nutzen. Dadurch könnten Prognosen und kontinuierliche Beobachtung von systemischen Therapien ausgesprochen bzw. durchgeführt werden. Weiterhin könnte die Charakterisierung und Bewertung von CTCs als diagnostisches Instrument genutzt werden, um eine geeignete Behandlung für solide Tumoren auszuwählen.

Für die Früherkennung, Diagnose und Therapiekontrolle von Krebs hat der Nachweis von zirkulierenden Tumorzellen (CTC) im Blut eine immer größer werdende Bedeutung. Dabei ist auf Grund der geringen Zahl an CTCs, die im Bereich von nur 3-5 in einem Milliliter Blut liegen kann und auf Grund des großen Hintergrundes an Leukocyten (6-10x10⁶ pro Milliliter) ein Verfahren zu wählen, das CTCs möglichst selektiv anzureichern oder aber vor einem großen Überschuss anderer Blutzellen darzustellen vermag. Zum Einsatz kommen dabei z.B. physikalische Methoden wie das Filtrieren, das mittels entsprechender Porengrößen eine Größenselektion der Zellen erlaubt, oder andere Verfahren, die z.B. über eine selektive Antikörper-Bindung die Anreicherung von CTCs in einer Blutprobe erlauben.

Kein Verfahren, das auf einer Größenselektion beruht, ist bislang vollautomatisiert. Zwischen dem Anreicherungsschritt und den eigentlichen selektiven immunochemischen Nachweisreaktionen der CTCs sind stets manuelle Arbeitsschritte notwendig.

Neben Flow-Cytometrischen Verfahren (z.B. das FACS Verfahren, fluorescence activated cell sorting) und Filtrationsverfahren ist das bislang einzig verfügbare kommerzielle für die Routine in-vitro-Diagnostik zugelassene System das der Firma Veridex (CellSearch). Das verwendete Verfahren erlaubt in einem Ausgangsvolumen von einigen ml Blut den Nachweis von CTCs, wenn deren nachzuweisende Zahl gleich oder über 3 pro Milliliter liegt. Das verwendete Verfahren zur Anreicherung nutzt die spezifische Bindung von an Magnetbeads gekoppelten Antikörpern an die CTCs. Der spezifische Nachweis der angereicherten CTCs erfolgt optisch mit Hilfe von Fluoreszenz markierten Antikörpern.

Es sind auch physikalische Verfahren bekannt, die über eine Grössenselektion die CTCs anreichern bzw. die Leukozyten im Blut abreichern und schonend "fixieren". Dabei kommen Filter mit definierten Porengrößen zum Einsatz, die die Permeation von Leukozyten und anderen Blutbestandteilen erlauben, jedoch ein Durchschlüpfen von CTCs verhindern sollen. Für die anschließende Prozessierung, die aus einer Reihe von Wasch- und selektiven Färbeschritt besteht, ist der manuelle Transfer des Filters auf eine Färbestation die übliche Vorgehensweise.

Demgegenüber ermöglicht die vorliegende Erfindung ein zuverlässiges, kostengünstiges automatisierbares Verfahren zum Nachweis von (lebenden) Zellen in einer Probe, insbesondere von Tumorzellen in einer Blutprobe.

### Beschreibung der Erfindung

Die Erfindung betrifft das Verfahren nach Anspruch 1 und die Anordnung nach Anspruch 8 .

Bei einem Filtrationsvorgang im Sinne der Erfindung wird eine Suspension durch ein Filter, z.B. eine Filtermembran, filtriert. Dabei wird Permeat durch den Filter gedrückt und Retentat auf der Filteroberfläche(oder auch in den Poren und Hohlräumen des Filters) zurückgehalten. Bei dem Filtriervorgang gibt es daher eine vorherrschende Strömungsrichtung des Permeats durch den Filter, so dass man von einem Bereich stromaufwärts von dem Filter sprechen kann, in welchem das Retentat (welches als wesentlichen Bestandteil die Zellen enthält) zurückgehalten wird, und einem Bereich stromabwärts, in welchen das Permeat durchgedrückt wird und z.b. dort gesammelt werden kann. Unabhängig von dieser vorherrschenden Strömungsrichtung kann in Ausnahmefällen die Strömungsrichtung auch umgekehrt werden, z.B. bei einer Rückspülung des Filters.

Um das Permeat durch den Filter zu drücken, kann eine Druckdifferenz erzeugt werden, wobei dann stromaufwärts von dem Filter ein höherer Druck vorliegt als Stromabwärts. Dies kann erreicht werden durch Anlegen eines Überdrucks stromaufwärts von dem Filter, Anlegen eines Unterdurcks stromabwärts oder einer Kombination aus beiden. Um den Permeatfluss durch den Filter zu stoppen (auf Null zu reduzieren), kann eine Druckdifferenz von Null eingestellt werden. Dies ist unabhängig von der Orientierung des Filters im Raum. Für den Sonderfall, dass die Strömungsrichtung am Filter vertikal verläuft oder eine vertikale Komponente (also in Richtung oder entgegen der Erdanziehungskraft) verläuft, ist ferner zu berücksichtigen, dass die Wassersäule auf dem Filter zur Druckkdifferenz beiträgt.

Für manche Anwendungen des erfindungsgemäßen Verfahrens ist es bevorzugt, dass die strömungsrichtung der Filtration am Filter im Wesentlichen in Richtung der Erdanziehungskraft verläuft. Dadurch kommt zurückgehaltenes Retentat auf der Oberfläche des Filters zu liegen, was z.b. eine einfache Weiterverarbeitung des Retenats (der Zellen) ermöglicht.

Für bestimmte Anwendungen kann es bevorzugt sein, nicht in Richtung der Erdanziehungskraft, sondern entgegen der Erdanziehungskraft zu Filtrieren, z.B. wenn das Retentat aufschwimmt, oder um Zellen nach dem Filtrieren von der Unterseite des Filters in einem Auffanggefäß zu sammeln.

Die Erfindung betrifft ein Verfahren zum Nachweis von Zellen in einer flüssigen Probe, aufweisend folgende Schritte:
a) Filtrieren der flüssigen Probe durch eine poröse Membran, die geeignet ist, nachzuweisende Zellen zurückzuhalten, derart, dass nachzuweisende Zellen auf zumindest einem Teil der Oberfläche der Membran zurückgehalten werden und dass zumindest ein Teil der Probenflüssigkeit die als Permeat Membran passiert,
b) Aufbringen einer ersten Prozessflüssigkeit, welche ein erstes Mittel zum Markieren der nachzuweisenden Zellen enthält,
c) Inkubieren der ersten Prozessflüssigkeit auf der Membran für einen vorbestimmten Zeitraum, wobei die nachzuweisenden Zellen markiert werden,
d) Detektieren der markierten nachzuweisenden Zellen auf der Oberfläche der Membran,
   wobei während Schritt c) ein Überdruck auf der Seite der Membran, welche der Seite abgewandt ist, auf der die nachzuweisenden Zellen in Schritt a) zurück gehalten worden sind, angelegt wird,
   und wobei der Überdruck so gewählt ist, dass ein Durchfluss von Prozessflüssigkeit durch die Membran hindurch verhindert wird.

Die flüssige Probe kann z.B. ein Blutprobe, Urinprobe oder Plasmaprobe sein.

Gemäß einer Ausführungsform der Erfindung wird Schritt b) realisiert, indem die Prozessfüssigkeit der flüssigen Probe direkt zugesetzt wird.

Gemäß einer Ausführungsform der Erfindung wird die erste Prozessflüssigkeit vor dem Detektieren der markierten nachzuweisenden Zellen entfernt, z.B. durch Durchdrücken durch die Membran.

Geeignete Mittel zum Markieren umfassen Marker die unspezifisch oder spezifisch Zellen anfärben können. Unspezifische Marker können z.B. Farbstoffe sein, die Proteine, Nukleinsäuren oder andere Zellbestandteile anfärben. Spezifische Marker können z.B. Antikörper, Sondenoligonucleotide, Peptide oder andere Moleküle sein, die spezifisch an Proteine, Nukleinsäuresequenzen oder andere Zellspezifische Strukturen binden. Der Marker kann mit einer nachweisbaren Markierung direkt markiert sein, z.B. chromogene Farbstoffe, Fluoreszenzfarbstoffe, Isotopenmarkierung o.ä.. Alternativ kann der Marker auch über ein sekundäres Nachweisreagenz (z.B. Sekundärantiköper oder Enzym-Substrat-System) nachgewiesen werden.

Dabei kann optional mindestens eine weitere Prozessflüssigkeit aufgebracht werden, z.B. vor Schritt b) oder nach Schritt d).

Vorzugsweise enthält die mindestens eine weitere Prozessflüssigkeit Mittel, die gewählt sind aus:
- Mitteln zum Waschen,
- Mitteln zum Fixieren von biologischen Strukturen,
- Mitteln zum Verhindern von unspezifischen Markierungsereignissen oder
- Mitteln zum Markieren der nachzuweisenden Zellen mit einem weiteren Marker gewählt sind, wobei sich der weitere Marker von dem ersten Mittel zum Markieren unterscheidet.

Als Mittel zum Waschen sind entsprechende Waschpuffer bekannt. Sie können ferner Mittel zur Permeabiliserung von Zellmembranen enthalten, z.B. Tenside, Saponin und ähnliche. Als Mittel zum Fixieren von biologischen Strukturen sind entsprechende Fixierlösungen bekannt, die Fixiermittel enthalten, z.B. Formalin, Glutaraldehyd und ähnliche.

Als Mittel zum Verhindern von unspezifischen Markierungsereignissen sind entsprechende Blockierpuffer bekannt. Wenn z.B. ein Antikörper als Marker verwendet wird, ist es bekannt, unspezifische Bindungen des Antikörpers durch einen Blockierpuffer abzusättigen, welcher unspezifische Immunglobuline der gleichen Spezies enthält, aus welcher der Marker-Antikörper stammt.

Bevorzugt enthält die weitere Prozessflüssigkeit Mittel, die zum Waschen oder Fixieren der Zellen oder zum Verhindern von unspezifischen Markierungsereignissen geeignet sind.

Gemäß einem bevorzugten Aspekt der Erfindung werden bei dem Verfahren nach dem Filtrieren die Zellen auf der Membran mit einem Farbstoff durch Inkubation mit einer entsprechenden Prozessflüssigkeit angefärbt. Dazu können Farbstoffe gewählt werden, die Zellen oder Zellbestandteile anfärben und aus Cytologie und Histologie bekannt sind. Dies können Lebend- oder Totfarbstoffe sein, Farbstoffe, die spezifisch Zellkerne oder andere Organellen anfärben oder die spezifisch bestimmte Zellkomponenten, z.B. Nukleinsäuren oder Proteine anfärben. Bekannte Zellfarbstoffe sind, z.B. Trypanblau, DAPI und ähnliche.

Die nachzuweisenden Zellen können auf der Membran ungefärbt oder gefärbt auch mikroskopisch analysiert werden. Erfindungsgemäß wird während Schritt c) ein Überdruck auf der Seite der Membran angelegt, welche der Seite abgewandt ist, auf der die nachzuweisenden Zellen in Schritt a) zurück gehalten worden sind, wobei der Überdruck so gewählt ist, dass ein Durchfluß von Prozessflüssigkeit durch die Membran hindurch verhindert wird, wobei der Überdruck ≤ 50mbar, ≤ 20mbar, ≤ 10mbar oder ≤ 5mbar beträgt. Bevorzugt beträgt der Überdruck 3-10 mbar. Durch einen geringen Überdruck wird verhindert, dass die Prozessflüssigkeit durch die Membran sickert.

Bevorzugt ist der Überdruck so gewählt, dass bei einer Strömungsrichtung nach unten (in Richtung der Erdanziehungskraft) gleichgroß oder größer als der Druck der Wassersäule über dem Filter ist. Dabei gilt: 1 cm Wassersäule entspricht ca. 1 mbar. Die Wassersäule entspricht der Füllstandshöhe der Suspension über dem Filter bei einer im wesentlichen horizontalen Anordnung der Filters, wobei von oben nach unten gefiltert wird.

Gemäß einer bevorzugten Ausführungsform der Erfindung wird während Schritt d) ein Unterdruck auf der Seite der Membran angelegt, welche der Seite abgewandt ist, auf der die nachzuweisenden Zellen in Schritt a) zu liegen gekommen sind, wobei der Unterdruck so gewählt ist, dass die Prozessflüssigkeit durch die Membran hindurch abgesaugt wird.

Gemäß einer bevorzugten Ausführungsform der Erfindung ist die nachzuweisende Zelle eine Tumorzelle. Das erfindungsgemäße Verfahren ist besonders gut für den Nachweis von CTCs geeignet.

Gemäß einer bevorzugten Ausführungsform der Erfindung sind in Schritt c) die Prozessflüssigkeit und die Inkubationsbedingungen so gewählt, dass ein Überleben der nachzuweisenden Zellen über den vorbestimmten Zeitraum zu ermöglicht wird. Dadurch ist es möglich an den Zellen weitere funktionelle Tests durchzuführen. Als Prozessflüssigkeit können hier Puffer mit physiologischen Salzkonzentrationen gewählt werden. Die Inkubation kann bei entsprechenden Temparatur- und Feuchtigkeitsbedingungen erfolgen.

Ferner können von den nachzuweisenden Zellen abgegebene Stoffe nachgewiesen werden.

Ferner betrifft die Erfindung eine Anordnung zur Durchführung der Schritte a) bis d) des erfindungsgemäßen Verfahrens, aufweisend:
a) eine poröse Membran, die geeignet ist, nachzuweisende Zellen zurückzuhalten
b) Mittel zum Aufbringen einer Flüssigkeit auf die Membran,
c) Mittel zum Durchdrücken der Flüssigkeit durch die Membran,
d) Mittel zum Einstellen einer Druckdifferenz vor und hinter der Membran,
e) Mittel zum Steuern des zeitlichen Ablaufs, die das Aufbringen der Flüssigkeit auf die Membran, das Verweilen der Flüssigkeit auf der Membran für eine vorbestimmte Zeitdauer und das Durchdrücken der Flüssigkeit durch die Membran steuern können,.
wobei die Mittel zum Einstellen der Druckdifferenz ermöglichen, einen Überdruck auf der Rückseite der Membran zu Erzeugen, um das Verweilen der Flüssigkeit auf der Membran für eine vorbestimmte Zeitdauer zu ermöglichen.

Die Mittel zum Aufbringen einer Flüssigkeit auf die Membran können z.B. als Zuleitung oder als Pippetiereinrichtung ausgeführt sein.

Die Mittel zum Durchdrücken der Flüssigkeit durch die Membran können Mittel umfassen, mit denen eine Druckdifferenz erzeugt wird, wobei dann stromaufwärts von dem Filter ein höherer Druck vorliegt als Stromabwärts.

Die Mittel zum Steuern des zeitlichen Ablaufs können als eine programmierbare Steuerung der Anordnung vorgesehen sein, bei der sich die vorbestimmte Zeitdauer einstellen lässt.

Gemäß einer bevorzugten Ausführungsform der Erfindung wird ein Überdruck auf der Seite der Membran angelegt, welche der Seite abgewandt ist, auf der die nachzuweisenden Zellen in zurück gehalten worden sind, wobei der Überdruck so gewählt ist, dass ein Durchfluß von Prozessflüssigkeit durch die Membran hindurch verhindert wird, wobei der Überdruck ≤ 50mbar, ≤ 20mbar, ≤ 10mbar oder ≤ 5mbar beträgt. Bevorzugt beträgt der Überdruck 3-10 mbar. Durch einen geringen Überdruck wird verhindert, dass die Prozessflüssigkeit durch die Membran sickert.

Bevorzugt ist der Überdruck so gewählt, dass bei einer Strömungsrichtung nach unten (in Richtung der Erdanziehungskraft) gleichgroß oder größer als der Druck der Wassersäule über dem Filter ist. Dabei gilt: 1 cm Wassersäule entspricht ca. 1 mbar. Die Wassersäule entspricht der Füllstandshöhe der Suspension über dem Filter bei einer im wesentlichen horizontalen Anordnung der Filters, wobei von oben nach unten gefiltert wird.

Die Membran kann in eine entsprechende Haltevorrichtung eingespannt sein, so dass entsprechende Unter- oder Überdrücke angelegt werden können.

Bevorzugt ist die Membran auf einem Objektträger angeordnet, so dass nach Durchführen der Schritte a) bis d) des erfindungsgemäßen Verfahrens der Objektträger mit der Membran zum Nachweis der Zellen begutachtet werden kann, z.B. durch Untersuchung mit einem Mikroskop.

Ein Mikoroskopie-Objektträger ist eine transparente ein Platte von ca. 26 x 76 mm (ISO 8255-2) und einer Dicke von ca. 0,1 bis 1.5 mm.

Der Objektträger weist bevorzugt Öffnungen auf, so dass die Flüssigkeiten problemlos abgesaugt werden können. Dies können mehrere kleine Öffnungen sein, z.B. Poren oder Bohrungen. Alternativ können es auch ein oder mehrere größere Ausschnitte sein, die von der Membran bedeckt werden können.

Die Membran weist z.B. eine Porengröße von 0,1 bis 200 µm auf. Dadurch können Zellen zurückgehalten werden, während Zellfragmente, Thrombozyten und kleinere Festbestandteile der Probe durch das Filter (die Membran) hindurch gehen.

Gemäß einem bevorzugten Aspekt der Erfindung weist die Membran eine Porengröße von 2 bis 50 µm, stärker bevorzugt 5 bis 20 µm, noch stärker bevorzugt 5 bis 10 µm, auf. Porengrößen der Größenbereiche 2 bis 50 µm, 5 bis 20 µm oder insbesondere 5 bis 10 µm bieten den Vorteil, dass die Zellen davon zurückgehalten werden, jedoch teilweise in den Poren haften bleiben und so besonders gut auf der Membran haften und für weitere Analysen zur Verfügung stehen.

Gemäß einem Aspekt der Erfindung wird mit dem ersten Marker ein Antigen aus der folgenden Liste 1 oder Liste 2 nachgewiesen.

Gemäß einem bevorzugten Aspekt der Erfindung wird bei Verwendung einer Blutprobe vor dem Filtrieren eine Erythrozytenlyse (z.B. durch hypotone Lyse) durchgeführt, um störende Erythrozyten zu entfernen.

Ferner können die Zellen auch nach dem Filtrieren wieder in Zellkulturmedium aufgenommen werden und für weitere Untersuchungen kultiviert werden. So können z.B. nachgewiesene Tumorzellen kultiviert und weiter untersucht werden, um das Ansprechen auf bestimmte Arzneimittel (z.B. Zytostatika) zu überprüfen.

Liste 1: bevorzugte zellspezifische Antigene:
- Alpha-l-Fetoprotein (AFP) bei Leberzellkarzinom und gonadalen und extragonadalen Keimzelltumoren
- Bence-Jones-Protein beim Multiplen Myelom
- Beta-HCG (beta-Untereinheit des humanen Choriongonadotropin) bei Keimzelltumoren des Ovars und nicht-seminomatösem Tumoren des Hodens
- CA 15-3 beim Brustkrebs (Mammakarzinom) oder Eierstockkrebs (Ovarialkarzinom)
- CA 19-9 und CA 50 beim Bauchspeicheldrüsenkrebs (Pankreaskarzinom)
- CA-125 beim Eierstockkrebs (Ovarialkarzinom)
- Calcitonin (humanes Calcitonin, hCT), beim medullären Schilddrüsenkarzinom
- Carcino-Embryonales Antigen (CEA) bei Darmkrebs, Pankreaskarzinom sowie Adenokarzinom der Lunge
- Cytokeratin-21-Fragment (CYFRA 21-1) und Serpin B4 (SCC) bei allen Varianten des Lungenkrebses (Bronchialkarzinoms)
- HER-2/neu
- HPV-Antikörper bzw. HPV-Antigene
- Homovanillinsäure beim Neuroblastom
- 5-Hydroxyindolessigsäure beim Karzinoid
- Katecholamine, Vanillinmandelsäure beim Phäochromozytom
- Laktat-Dehydrogenase (LDH) bei Keimzelltumoren
- Laktat-Dehydrogenase Isoenzym 1 (LDH-1) bei Keimzelltumoren; eine routinemäßige Bestimmung wird in den gängigen Leitlinien jedoch noch nicht empfohlen
- MAGE Antigene
- Metanephrine beim Phäochromozytom
- MUC1 beim nicht kleinzelligen Bronchialkarzinom (NSCLC) oder beim Mammakarzinom
- NSE beim kleinzelligen Bronchialkarzinom (SCLC), Neuroblastom sowie seminomatösen Keimzelltumoren
- Plazentare alkalische Phosphatase (PLAP) bei seminomatösen Keimzelltumoren
- PSA beim Prostatakrebs (Prostatakarzinom)
- Thyreoglobulin (Tg) in jeder Konzentration beim papillären oder follikulären Schilddrüsenkarzinom
- Thymidinkinase
- Cytokeratine, z.B. Cytokeratin 8, 18, 19

Liste 2: Zusätzliche zellspezifische Antigene
- β2-Mikroglobulin (β2-M),
- CA 54-9,
- CA 72-4,
- CA 195,
- Cancer Associated Serum Antigen (CASA),
- C-Peptid,
- Cytokeratin,
- Gastrin,
- Glucagon,
- Glucose-6-phosphat-Isomerase (GPI),
- Insulin,
- Neopterin,
- nukleäres Matrixprotein 22 (NMP 22),
- Ostase,
- P53-Autoantikörper,
- Paraproteine,
- Prolaktin (PRL),
- Protein S-100,
- Serpin B4 (SCC),
- Schwangerschaftsspezifisches β1-Glykoprotein (SP-1),
- Tumor-assoziiertes Glykoprotein 12 (TAG 12),
- Thymidinkinase (TK),
- Tissue polypeptide antigen (TPA),
- Tissue polypeptide specific antigen (TPS),
- Tumor M2-PK,
- Vasoaktives intestinales Polypeptid (VIP),
- Transketolase-like-l-Protein (TKTL1)

Die in Liste 1 und 2 genannten Antigene sind beispielhafte Targets für spezifische Marker (z.B. Antikörper). Über welche gemäß dem erfindungsgemäßen Verfahren Zellen, insbesondere Tumorzellen nachgewiesen werden können.

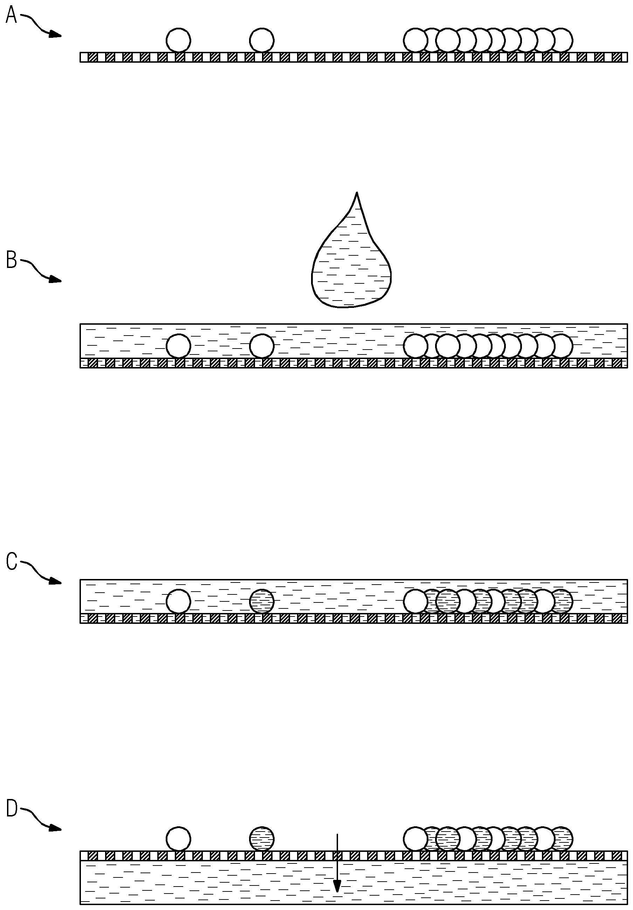

Das Erfindungsgemäße Verfahren wird im Folgenden beispielhaft beschrieben. In der angehängten Figur 1 sind die Schritte a) bis d) des erfindungsgemäßen Verfahrens schematisch dargestellt.
Schritt A zeigt das Filtrieren der flüssigen Probe durch eine poröse Membran derart, dass nachzuweisende Zellen auf der Oberfläche der Membran zurückgehalten werden bzw. zu liegen kommen,
Schritt B zeigt das Aufbringen einer ersten Prozessflüssigkeit, welche ein erstes Mittel zum Markieren der nachzuweisenden Zellen enthält,
Schritt C zeigt das Inkubieren der Prozessflüssigkeit auf der Membran für einen vorbestimmten Zeitraum, wobei die nachzuweisenden Zellen markiert werden (durch Schraffierung der markierten Zellen dargestellt), und
Schritt D zeigt das Entfernen der Prozessflüssigkeit, z.B. durch Absaugen.

Die von dem umgebenden Medium (Probenflüssigkeit) zu trennenden Feststoffe (Zelle, Partikel, Gewebe) werden vom Medium dadurch getrennt, dass Sie auf der Oberfläche einer Filtermembran verbleiben, die impermeabel für die zu trennenden Feststoffe ist, jedoch permeabel für das Umgebungsmedium und auch für den zu trennenden Feststoff verunreinigende Feststoffe ist. Die dazu sind Mittel vorgesehen, die es erlauben, die Flussrate des Gases oder der Flüssigkeit durch die für sie permeable Oberfläche gezielt zu bestimmen und zu verändern. Vorzugsweise handelt sich in der medizinischen Diagnostik bei den Feststoffen um zelluläre Komponenten und bei dem umgebenden Medium um Vollblut/Serum/Plasma, das auch Partikel enthalten kann, für die jedoch die Oberfläche auf Grund ihrer Eigenschaften permeabel ist (z.B. Leukozyten).

Durch eine automatisierte Vorgehensweise werden neben der Probe alle für die Isolierung/Anreicherung und den Nachweis notwendigen Reagenzien gezielt auf die Oberfläche der Membran aufgebracht, beispielsweise durch einen Pipettier-Roboter. Die Probe sowie die Reagenzien können kontrolliert auf der Oberfläche zu Inkubationszwecken verbleiben und/oder durch die für sie permeable Oberfläche entfernt werden. Dies wird durch die Verwendung verschiedener Sensoren und Aktuatoren erreicht, die eine je nach den Erfordernissen geregelten Durchtritt der Stoffe durch die Permeationsschicht (permeable Oberfläche) der Membran erlauben. Dabei kann die Regelung beispielsweise nach der Flussrate oder aber auch, bei besonders empfindlichen zellulären Komponenten, mittels einer am System angelegten Druckdifferenz (zum Umgebungsdruck) erfolgen.

Sämtliche Schritte, vom Aufbringen des Mediums auf die semipermeable Oberfläche bis zur selektiven Markierung werden vorzugsweise in einem Liquid Handling Roboter automatisch durchgeführt und überwacht. Dabei kann eine gleichzeitige, parallele Bearbeitung einer Mehrzahl von zu untersuchenden Proben erreicht werden.

Durch die Abarbeitung des sequenziellen Isolierung/Anreicherungs-Protokolls und der selektiven Nachweisreaktionen in einer Geräte-Einheit, ohne die bislang üblichen manuellen Zwischenschritte und den Probentransfer wird eine weniger fehleranfällige Analyse und eine schnellere Durchlaufzeit erreicht. Da bei dem hier vorgeschlagenen Verfahren auch die Inkubation aller für den Nachweis notwendiger Prozessflüssigkeiten bzw. Reagenzien mittels gezieltem Aufbringen auf die semipermeable Oberfläche und den gesteuerten Fluss durch diese Oberfläche geregelt wird, lassen sich die eingesetzten Reagenzienvolumina deutlich verringern, was bei den oft hochpreisigen immunochemischen Reagenzien einen erheblichen Kostenvorteil bedeutet. Sowohl beim manuellen immunochemischen Färben als auch bei den bekannten "Autostainern" ist das Inkubationsvolumen deutlich größer, da nicht nur die zu behandelnde Oberfläche benetzt werden muss, sondern eine sehr viel größere Fläche überspült wird bzw. das gesamte Inkubationsgefäß mit einem ausreichenden Volumen befüllt werden muss.

Für die Durchführung der Anreicherung-/Wasch- und Färbeschritte ist es notwendig:
(a) das Flüssigkeitsvolumen bzw. den Füllstand über der semipermeablen Oberfläche
(b) die Geschwindigkeit der Permeation der Flüssigkeit durch die semipermeable Oberfläche
(c) die für die Permeation notwendige Druckdifferenz zwischen der Umgebung und der Apparatur zu kennen und zur Steuerung der Permeationsgeschwindigkeit und zur Bestimmung des aktuell analysierten Probe¬volumens zu nutzen.

Die dafür notwendige Sensorik und Aktorik kann sowohl Bestandteil der Permeations-Apparatur als auch des Pipettier-Roboters sein oder aber verteilt vorliegen, was eine Kommunikation zwischen den Komponenten erforderlich macht.

Für die individuelle Ansteuerung von jeder/jedem einzelnen Probe/Kanal wird mindestens 1 Drucksensor und 1 Ventil benötigt, mit dem sich die am System anliegende Druckdifferenz zur Umgebung (also der Überdruck bzw. Unterdruck) regeln lässt. Die jeweilige Druckdifferenz wird beispielsweise durch die Verbindung mit einem Vorratsgefäß hergestellt, in dem entweder ein Unter- oder ein Überdruck gegenüber der Umgebung herrscht.

Eine Überwachung des Durchflusses an Flüssigkeit kann vorteilhafterweise durch einen Liquid Handling Roboter erfolgen, der dazu folgende Schritte übernimmt:
(a) Kanalspezifische Füllstandsmessung (z.B. kapazitiv) zur Bestimmung des Flüssigkeitsvolumens über der semipermeablen Membran zur Überlaufkontrolle und Steuerung des Zeitpunkts des Nachpipettierens,
(b) Umwandlung der erfassten Meßdaten in Daten, die von einer externen Software gelesen und einer weiteren Verarbeitung zugeführt werden können,
(c) in Abhängigkeit vom jeweiligen Füllstand das Zupipettieren eines weiteren Flüssigkeitsaliquots.

Die Geschwindigkeit, mit der die Permeation des Mediums durch die semipermeable Oberfläche erfolgt, wird vorteilhafterweise entweder über die Flussrate oder die zur Permeation notwendige Druckdifferenz gesteuert. Dazu wird bei empfindlichen zu isolierenden Komponenten eine möglichst geringe Druckdifferenz z.B. von bis zu 7 mbar angelegt, diese kann aber auch auf bis zu 50 mbar ansteigen. Die fortlaufende Messung der Druckdifferenz dient aber auch zur Feststellung, ob sich noch Probenmaterial über der semipermeablen Membran befindet, und kann somit als Signal für die beendete Permeation dienen, oder aber als Analyse-Abschaltsignal genutzt werden, falls es während des Assay-Ablaufs zu einer Verstopfung der semipermeablen Oberfläche kommt.

Durch portioniertes Zugeben der Probe, z.B. durch einen Liquid-handling-Roboter, und gelegentliches Aufmischen der Probe, kann eine Sedimentation der Zellen und damit einhergehend ein Zellverlust weitestgehend vermieden werden.

Beispielhaft beschrieben werden die automatisierten Schritte für die Anreicherung und den immunochemischen Nachweis bzw. die moleklarbiologische Charakterisierung von CTCs in einer Blutprobe.
(i) Manuelle Arbeitsschritte:
   - Einsetzen der zu untersuchenden Blutproben in das Gerät
   - Einsetzen der benötigten vorbereiteten Reagenzien
   - Einsetzen des sonstigen Verbrauchsmaterials (z.B. Pipettenspitzen)
   - Nach Analyse: Entfernen des Abfalls
(ii) Ablauf der automatisierten CTC Anreicherung und immunochemischer CTC Nachweis:
   - Mischen der Blutproben
   - Entnahme der Blutprobe und Zugabe zu einem Verdünnungs-/Aufschluss-/Fixierungs-Puffer
   - Inkubation von Blutprobe und Puffer
   - wahlweise Konditionierung der Membran durch eine Reihe von Wasch- und Inkubationsschritten
   - Pipettieren der behandelten Blutprobe in die Anordnung, so, dass die Membran bedeckt wird.
   - Durch Füllstands- und Druckdifferenz gesteuerte Anreicherung der CTCs auf der Membran (z.B. durch Füllstandssensorik in der Pipettenspitze, Druckmessung und Steuerung der Druckdifferenz mittels Ventil zum Unter-/Überdruckvorratsgefäss)
   - Abschaltung des Permeationsvorgangs bei dauerhaftem Zusetzen der Membran
   - Mildes Fixieren und Waschen der Zellen auf der semipermeablen Membran
   - Immunochemisches Färben durch Inkubation der Zellen auf der Membran mit den notwendigen Prozessflüssigkeiten /Reagenzien
   - Durch Füllstands- und Druckdifferenz gesteuerter Assayablauf (Füllstandssensorik in der Pipettenspitze, Druckmessung und Steuerung der Druckdifferenz mittels Ventil zum Unter-/Überdruckvorratsgefäss)
   - Abschaltung des Permeationsvorgangs bei dauerhaftem Zusetzen Membran
      Wahlweise nachgeschaltet: (c) "moleklarbiologische Charakterisierung" durch FISH (Fluroescence in-situ hybridization)
   - Molekularbiologisches Färben durch temperierte Inkubation auf der Membran mit den notwendigen Reagenzien
   - Verhinderung von Verdunstung bei längerer Inkubation durch Auflegen eines Deckglases, welches nach der Inkubation wieder entfernt wird
(iv) Abschließende Schritte
   - Pipettieren eines geeigneten Mountingmediums, welches auch verfestigend sein kann, auf die semipermeable Membran
   - Auflegen eines Deckglases

Schritt (iv) kann wahlweise wieder manuell erfolgen.

Beim sog. Immunostaining werden Körperzellen oder Zellverbände (Gewebeschnitte) z.B. mit Antikörperlösungen, Label-Reagenzien, Waschpuffer und vielerlei Prozessflüssigkeiten (zumeist sequentiell) inkubiert. Typischerweise werden die gefärbten Objekte auf einem Mikroskop-Objektträger (Slide) mit einem Mikroskop analysiert.

Die für das Anfärben (Staining) erforderlichen fluidischen Prozessschritte sind sehr vielfältig und komplex. Die verwendeten Reagenzien sind teilweise sehr kostspielig. Ein automatisiertes Prozessieren auf einem Objektträger lässt eine kostensparende Prozessierung mit wenig Volumen kaum zu. Die Reagenzien werden meist über den Objektträger hinweggespült, was neben einem hohen Verbrauch an Reagenzien u.U. auch mit einem teilweisen Verlust von Objekten einhergehen kann.

Erfindungsgemäß werden die Reagenzien nicht über eine Oberfläche hinweggespült, sondern durch eine Membran hindurchgespült, was Reagenzien einspart, den Verlust von Objekten vermeidet und eine generell bessere Prozesskontrolle erlaubt.

## Patentansprüche

1. Verfahren zum Nachweis von Zellen in einer flüssigen Probe, aufweisend folgende Schritte:
a) Filtrieren der flüssigen Probe durch eine poröse Membran durch Erzeugen einer Druckdifferenz, wobei stromaufwärts von dem Filter ein höherer Druck vorliegt als stromabwärts, wobei die poröse Membran geeignet ist, nachzuweisende Zellen zurückzuhalten, derart, dass nachzuweisende Zellen auf zumindest einem Teil der Oberfläche der Membran zu liegen kommen und dass zumindest ein Teil der Probenflüssigkeit die Membran passiert,
b) Aufbringen einer ersten Prozessflüssigkeit, welche ein erstes Mittel zum Markieren der nachzuweisenden Zellen mit einem ersten Marker enthält,
c) Inkubieren der Prozessflüssigkeit auf der Membran für einen vorbestimmten Zeitraum, wobei die nachzuweisenden Zellen markiert werden,
d) Detektieren der markierten nachzuweisenden Zellen auf der Oberfläche der Membran,
wobei während Schritt c) ein Überdruck auf der Seite der Membran anliegt, welche der Seite abgewandt ist, auf der die nachzuweisenden Zellen in Schritt a) zu liegen gekommen sind, wobei der Überdruck so gewählt ist, dass ein Durchfluß von Prozessflüssigkeit durch die Membran hindurch verhindert wird.

2. Verfahren nach Anspruch 1, wobei vor Schritt b) oder nach Schritt c) mindestens eine weitere Prozessflüssigkeit aufgebracht wird.

3. Verfahren nach Anspruch 1 oder 2, wobei die mindestens eine weitere Prozessflüssigkeit Mittel enthält, die aus Mitteln zum Waschen, Mitteln zum Fixieren von biologischen Strukturen, Mitteln zum Verhindern von unspezifischen Markierungsereignissen oder Mitteln zum Markieren der nachzuweisenden Zellen mit einem weiteren Marker gewählt sind, wobei sich der weitere Marker von dem ersten Marker unterscheidet.

4. Verfahren nach einem der vorangehenden Ansprüche, wobei die zweite Prozessflüssigkeit Mittel enthält, die zum Waschen oder Fixieren der Zellen oder zum Verhindern von unspezifischen Markierungsereignissen geeignet sind.

5. Verfahren nach einem der vorangehenden Ansprüche, wobei während Schritt d) ein Unterdruck auf der Seite der Membran anliegt, welche der Seite abgewandt ist, auf der die nachzuweisenden Zellen in Schritt a) zu liegen gekommen sind, wobei der Unterdruck so gewählt ist, dass die Prozessflüssigkeit durch die Membran hindurch abgesaugt wird.

6. Verfahren nach einem der vorangehenden Ansprüche, wobei die nachzuweisenden Zellen Tumorzellen sind.

7. Verfahren nach einem der vorangehenden Ansprüche, wobei von den nachzuweisenden Zellen abgegebene Stoffe nachgewiesen werden.

8. Anordnung zur Durchführung des Verfahrens nach einem der Ansprüche 1 bis 7, aufweisend:
a) eine poröse Membran, die geeignet ist, nachzuweisende Zellen zurückzuhalten,
b) Mittel zum Aufbringen einer Flüssigkeit auf die Membran,
c) Mittel zum Durchdrücken der Flüssigkeit durch die Membran,
d) Mittel zum Einstellen einer Druckdifferenz vor und hinter der Membran,
e) Mittel zum Steuern des zeitlichen Ablaufs, die das Aufbringen der Flüssigkeit auf die Membran, das Verweilen der Flüssigkeit auf der Membran für eine vorbestimmte Zeitdauer und das Durchdrücken der Flüssigkeit durch die Membran steuern kann,
wobei die Mittel zum Einstellen der Druckdifferenz ermöglichen, einen Überdruck von ≤ 50mbar auf der Rückseite der Membran zu erzeugen, um das Verweilen der Flüssigkeit auf der Membran für eine vorbestimmte Zeitdauer zu ermöglichen, und wobei die Membran auf einem Objektträger angeordnet ist.

9. Anordnung nach Anspruch 8, ferner aufweisend einen Abfallbehälter zum Sammeln von durch die Membran durchgedrückten Flüssigkeiten.

## Claims

1. Method for detecting cells in a liquid sample, having the following steps:
a) filtering the liquid sample through a porous membrane by generation of a pressure difference, in which case a higher pressure is present upstream of the filter than downstream, the porous membrane being suitable for retaining cells to be detected, such that cells to be detected come to rest on at least part of the surface of the membrane and at least some of the sample liquid passes through the membrane,
b) applying a first process liquid containing a first agent for marking the cells to be detected with a first marker,
c) incubating the process liquid on the membrane for a predetermined time period, wherein the cells to be detected are marked,
d) detecting the marked cells to be detected on the surface of the membrane,
where during step c) a superatmospheric pressure is present on the side of the membrane which faces away from the side on which the cells to be detected have come to rest in step a), the superatmospheric pressure being selected such that flow of process liquid through the membrane is prevented.

2. Method according to Claim 1, wherein before step b) or after step c) at least one further process liquid is applied.

3. Method according to Claim 1 or 2, wherein the at least one further process liquid contains agents which are selected from agents for washing, agents for fixing biological structures, agents for preventing nonspecific marking events or agents for marking the cells to be detected with a further marker, where the further marker differs from the first marker.

4. Method according to any of the preceding claims, wherein the second process liquid contains agents which are suitable for the washing or fixing of the cells or for the prevention of nonspecific marking events.

5. Method according to any of the preceding claims, wherein during step d) a subatmospheric pressure is present on the side of the membrane which faces away from the side on which the cells to be detected have come to rest in step a), the subatmospheric pressure being selected such that the process liquid is absorbed through the membrane.

6. Method according to any of the preceding claims, wherein the cells to be detected are tumour cells.

7. Method according to any of the preceding claims, wherein substances released from the cells to be detected are detected.

8. Array for carrying out the method according to any of Claims 1 to 7, having:
a) a porous membrane which is suitable for retaining cells to be detected,
b) means for applying a liquid to the membrane,
c) means for squeezing the liquid through the membrane,
d) means for establishing a pressure difference in front of and behind the membrane,
e) means for controlling the time progression which can control the application of the liquid to the membrane, the residence of the liquid on the membrane for a predetermined time period and the squeezing of the liquid through the membrane,
where the means for establishing the pressure difference permit a superatmospheric pressure of ≤ 50 mbar to be generated on the back of the membrane in order to permit the residence of the liquid on the membrane for a predetermined time period, and where the membrane is arranged on a microscope slide.

9. Array according to Claim 8, also having a waste container for collecting liquids squeezed through the membrane.

## Revendications

1. Procédé de détection de cellules dans un échantillon liquide, présentant les étapes suivantes consistant à :
a) filtrer l'échantillon liquide à travers une membrane poreuse en produisant une différence de pression, dans lequel une pression plus élevée se trouve en amont du filtre comme en aval, dans lequel la membrane poreuse est conçue pour retenir les cellules à détecter, de sorte que les cellules à détecter viennent se placer sur au moins une partie de la surface de la membrane et qu'au moins une partie de l'échantillon liquide traverse la membrane,
b) appliquer un premier liquide de processus, lequel contient un premier agent de marquage des cellules à détecter avec un premier marqueur,
c) incuber le liquide de processus sur la membrane pendant une durée prédéterminée, dans lequel les cellules à détecter sont marquées,
d) détecter les cellules à détecter marquées à la surface de la membrane,
dans lequel, pendant l'étape c), une surpression est appliquée sur le côté de la membrane, laquelle est opposée au côté, sur lequel les cellules à détecter à l'étape a) ont été placées, dans lequel la surpression est choisie de telle manière qu'un écoulement du liquide de processus à travers la membrane est empêché.

2. Procédé selon la revendication 1, dans lequel, avant l'étape b) ou après l'étape c), au moins un autre liquide de processus est appliqué.

3. Procédé selon la revendication 1 ou 2, dans lequel l'au moins un autre liquide de processus contient des agents, qui sont choisis parmi des agents de lavage, des agents de fixation de structures biologiques, des agents de prévention d'événements de marquages non spécifiques ou des agents de marquage des cellules à détecter à l'aide d'un marqueur supplémentaire, dans lequel le marqueur supplémentaire se différencie du premier marqueur.

4. Procédé selon l'une quelconque des revendications précédentes, dans lequel le second liquide de processus contient des agents, qui sont conçus pour laver ou fixer des cellules ou pour prévenir des d'événements de marquage non spécifiques.

5. Procédé selon l'une quelconque des revendications précédentes, dans lequel, pendant l'étape d), une dépression est appliquée sur le côté de la membrane, laquelle est opposée au côté sur lequel les cellules à détecter ont été placées à l'étape a), dans lequel la dépression est choisie de telle manière que le liquide de processus est aspiré à travers la membrane.

6. Procédé selon l'une quelconque des revendications précédentes, dans lequel les cellules à détecter sont des cellules tumorales.

7. Procédé selon l'une quelconque des revendications précédentes, dans lequel des substances libérées des cellules à détecter sont détectées.

8. Agencement pour la réalisation du procédé selon l'une quelconque des revendications 1 à 7, présentant :
a) une membrane poreuse, qui est conçue pour retenir les cellules à détecter,
b) un agent destiné à appliquer un liquide sur la membrane,
c) un agent destiné à faire passer le liquide à travers la membrane,
d) un agent destiné à régler une différence de pression devant et derrière la membrane,
e) un agent destiné à réguler l'écoulement dans le temps, qui peut réguler l'application du liquide sur la membrane, la présence du liquide sur la membrane pendant une durée prédéterminée et le passage du liquide à travers la membrane,
dans lequel les agents destinés à régler la différence de pression permettent de produire une surpression de ≤ 50 mbar sur le côté arrière de la membrane pour permettre la présence du liquide sur la membrane pendant une durée prédéterminée, et dans lequel la membrane est agencée sur une lamelle.

9. Agencement selon la revendication 8, présentant en outre un conteneur à déchets destiné à collecter les liquides passés à travers la membrane.
